# EUROPÄISCHE PATENTANMELDUNG

(11) **EP 2 535 011 A1**
(43) Veröffentlichungstag der Anmeldung: **19.12.2012**
(21) Anmeldenummer: 11004968.1
(22) Anmeldetag: 17.06.2011
(51) Int. Cl.: A61B 17/66

(54) **Einrichtung zur Distraktion eines Knochensegmentes**

(71) Anmelder: Krenkel, Christian, 5020 Salzburg (AT)
(72) Erfinder: Krenkel, Christian, 5020 Salzburg (AT)
(74) Vertreter: Hofinger, Stephan

(57) **Zusammenfassung**

Einrichtung zur Distraktion eines Knochensegmentes (3) von einem ortsständigen Knochen (4), mit einem verdrehbaren glatten Schaft (1), an den eine gerade Verankerungsschraube (2) anschließt, bei deren Herausschrauben ein Mitnehmer die Axialbewegung aber nicht die Drehbewegung auf das Knochensegment (3) überträgt, dadurch gekennzeichnet, dass als Mitnehmer für das Knochensegment (3) eine mit einem offenen Schlitz (13) versehene Druckplatte (6) vorgesehen ist, welche durch Einschieben orthogonal zur Längsrichtung des Schaftes (1) an diesem in Axialrichtung festlegbar ist.

## Beschreibung

Die Erfindung bezieht sich auf eine Einrichtung zur Distraktion eines Knochensegmentes von einem ortsständigen Knochen, mit einem verdrehbaren glatten Schaft, an den eine gerade Verankerungsschraube anschließt, bei deren Herausschrauben ein Mitnehmer die Axialbewegung aber nicht die Drehbewegung auf das Knochensegment überträgt.

Eine derartige Einrichtung, die sich in der Praxis bewährt hat, ist aus EP 1 259 177 B1 bekannt. Hinsichtlich der Zielsetzung und der grundsätzlichen Funktionsweise unterscheidet sich die Erfindung nicht von der dort beschriebenen Einrichtung, weshalb diesbezüglich auf die Ausführungen in EP 1 259 177 B1 verwiesen wird.

Der Mitnehmer, welcher bei der bekannten Einrichtung das Knochensegment vom ortsständigen Knochen wegdrückt, besteht dort aus einer den Schaft umgebenden Hülse, welche beim Herausschrauben der Verankerungsschraube durch einen, den Schaft umgebenden Ringflansch beaufschlagt wird. Um den entsprechenden Druck auf das Knochensegment übertragen zu können, ist die Hülse in dieses eingeschraubt. Nach Beendigung des Verfahrens muss sie dementsprechend durch Herausschrauben aus dem Knochensegment entfernt werden.

Die Erfindung vermeidet den aufgezeigten Nachteilt dadurch, dass als Mitnehmer für das Knochensegment eine mit einem offenen Schlitz versehene Druckplatte vorgesehen ist, welche durch Einschieben orthogonal zur Längsrichtung des Schaftes an diesem in Axialrichtung festlegbar ist.

Ein besonderer Vorteil der Erfindung liegt darin, dass sie es erlaubt, auf eine den Schaft umgebende Hülse ganz zu verzichten, womit sich auch das Problem der Abdichtung zwischen Hülse und Schaft erübrigt und somit eine wesentliche konstruktive Vereinfachung gegenüber dem Stand der Technik erreicht wird. Voraussetzung hiefür ist lediglich, dass der Durchmesser des Schaftes nicht kleiner ist als der der Verankerungsschraube. Dieses Merkmal ist notwendig, damit die Verankerungsschraube nach Beendigung des Verfahrens durch die den Schaft umschließende Bohrung entfernt werden kann.

Grundsätzlich könnte die axiale Festlegung der erfindungsgemäßen Druckplatte durch den Schaft umgebende Flansche, Splinte oder dergleichen erfolgen. Wenn auf die Verwendung einer den Schaft umgebenden Hülse verzichtet werden soll, muss dann wieder vorgesehen sein, dass dieser Flansch bzw. Splint orthogonal zur Längsrichtung des Schaftes eingesetzt werden kann, nachdem die Verankerungsschraube eingesetzt worden ist und vor der Entfernung wieder orthogonal entfernt werden kann. Ein solcher Flansch oder Splint wird vorzugsweise überhaupt vermieden, indem zur Festlegung der Druckplatte am Schaft eine umlaufende Rinne vorgesehen ist, in welche der Schlitz der Druckplatte eingreift.

Einzelheiten der Erfindung werden anschließend anhand der Zeichnungen erläutert. In dieser ist
- Fig. 1: eine schaubildliche Darstellung eines Ausführungsbeispiels der erfindungsgemäßen Einrichtung in Anwendung auf einen Oberkiefer,
- Fig. 2: ein Längsschnitt durch ein leicht modifiziertes Ausführungsbeispiel der Einrichtung nach Fig. 1 und
- Fig. 3a bis 3c: zeigen schaubildlich drei Varianten der den Kern der Erfindung bildenden Druckplatte 6 in Verbindung mit der den Schaft 1 umgebenden Rinne 9.

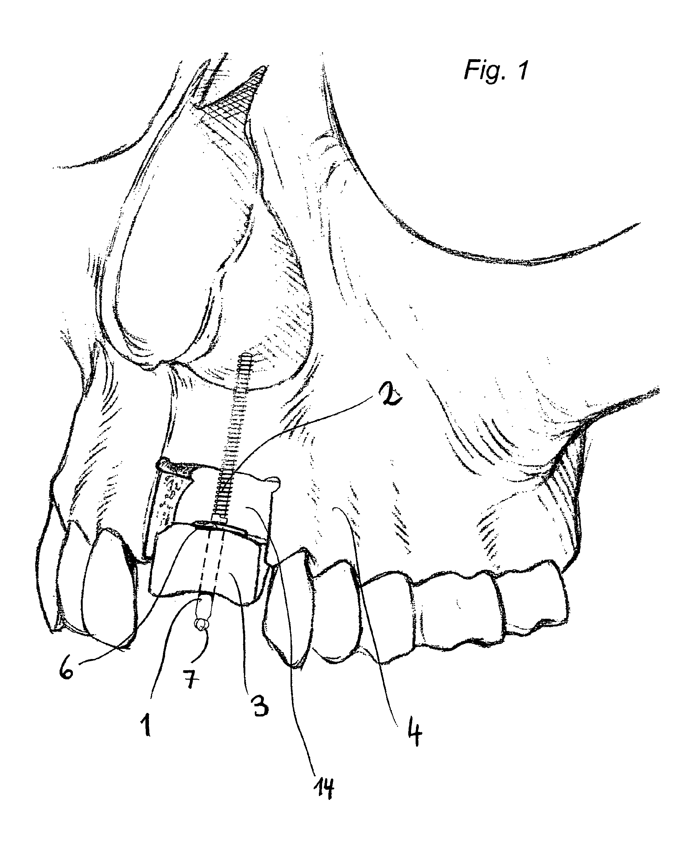

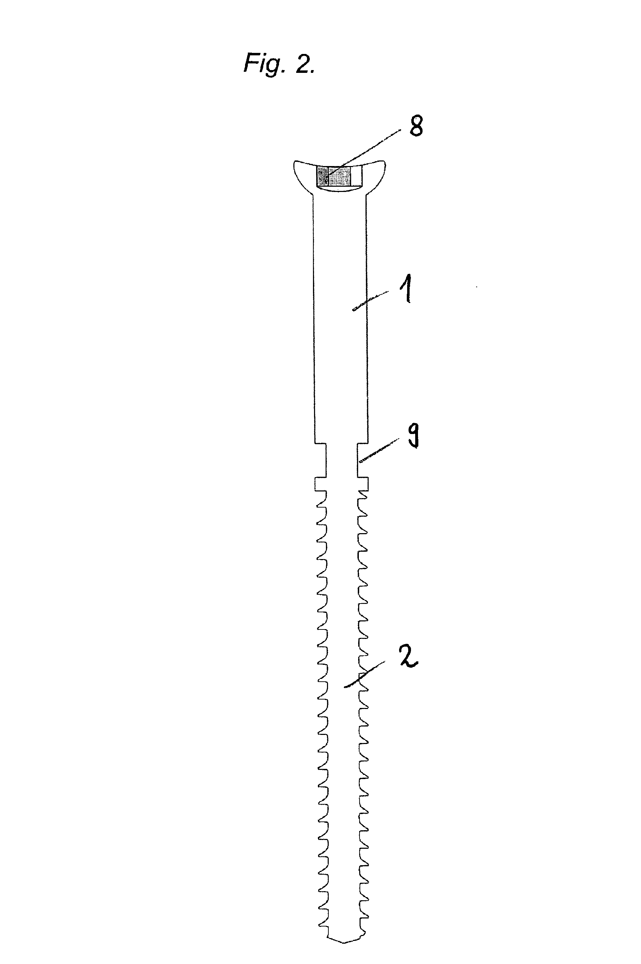

Die in Figur 1 dargestellte Einrichtung dient wie beim Stand der Technik dazu, zwischen einem Knochensegment 3 und dem ortsständigen Knochen 4 im Osteotomiespalt neues Gewebe aufzubauen. Hiezu wird dieser Spalt durch Verdrehen des Schaftes 1 und der Verankerungsschraube 2 aus der Grundstellung, in welcher lediglich ein geringfügiger Osteotomiespalt vorliegt, in die dargestellte Position gebracht, während sich in Spalt 14 allmählich neues Gewebe bildet. Die Verdrehung des Schaftes 1 samt der diesen einstückig fortsetzende Verankerungsschraube 3 erfolgt durch den Patienten selbst mittels des gerändelten Schraubenkopfes 7. Dies ist einfacher als die Verdrehung mittels Imbus, wie sie gemäß Figur 2 vorgesehen ist.

Eine Verdrehung des Knochensegmentes 3 wird durch die Seitenwände der Ausnehmung im Knochen 4, durch das straffe durchgehende Zahnfleisch am Kieferkamm und durch das neu gebildete Gewebe verhindert. Bei großen Knochensegmenten, welche durch zwei parallele Einrichtungen distrahiert werden, verhindert ohnedies die jeweils andere Einheit eine Rotationsbewegung.

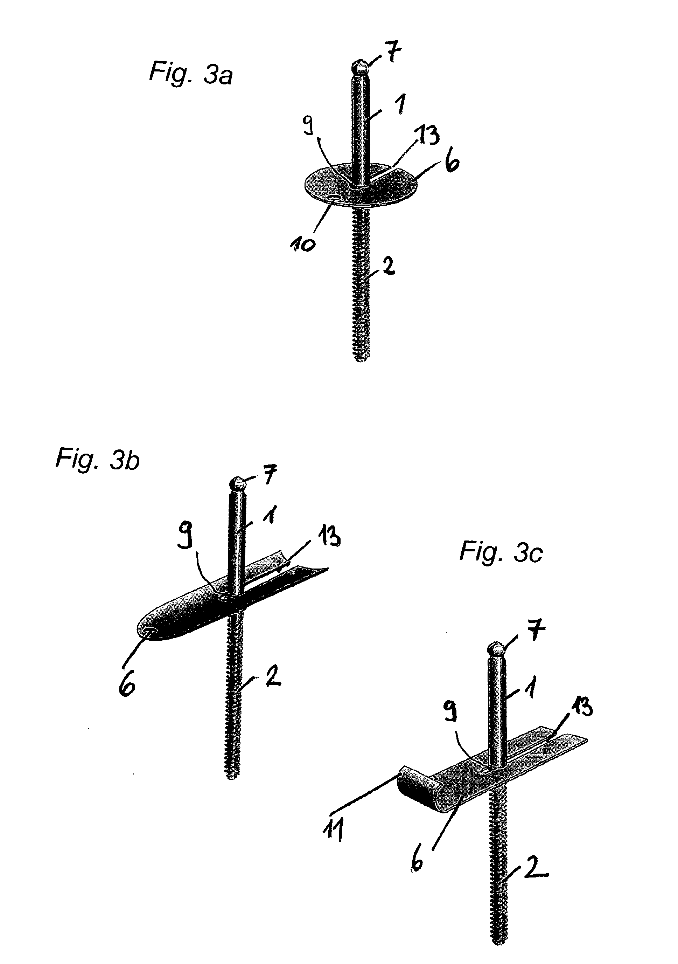

Zur Übertragung der Längsbewegung der Verankerungsschraube 2 auf das Knochensegment 3 dient erfindungsgemäß eine Druckplatte 6. Deren Festlegung am Schaft 1 erfolgt, wie aus Figur 2 hervorgeht, durch eine umlaufende Rinne 9 am Schaft 1. Die äußere Form der Druckplatte 6 kann, wie aus Figur 3 ersichtlich, durchaus variieren. Wesentlich ist der offene Schlitz 13, dessen Breite dem Durchmesser des Schaftes im Bereich der Rinne 9 entspricht, sodass die sich am Knochensegment 3 abstützende Druckplatte 6 die Axialbewegung des Schaftes 1 mitmacht, wenn die Verankerungsschraube 2 im Basisknochen mittels des Schraubenkopfes 7 herausgeschraubt wird.

Figur 3a zeigt die Grundform der Druckplatte 6, welche am Beginn der Behandlung nach Einsetzen der Verankerungsschraube 2 orthogonal implantiert wird. Um die Entfernung der Druckplatte 6 nach Abschluss des Verfahrens zu erleichtern, ist diese mit einer Bohrung versehen. Die Druckplatte 6 kann länglich ausgestaltet sein, wie dies in Figur 3b dargestellt ist, falls dies erforderlich ist, um sie bis zum Rand des Knochensegmentes 3 zu führen. Eine derartige längliche Druckplatte 6 sollte allerdings verlässlich daran gehindert werden, die Drehung der Verankerungsschraube 2 mitzumachen. Zu diesem Zweck kann sie etwa wie in Figur 3c dargestellt, mit einem aufgebogenen Rand 11 versehen werden, welcher sie am Knochensegment 3 festhält.

Eine Entfernung der Druckplatte 6 nach Beendigung der Behandlung kann unterbleiben, wenn diese aus resorbierbarem Material gefertigt ist.

## Patentansprüche

1. Einrichtung zur Distraktion eines Knochensegmentes (3) von einem ortsständigen Knochen (4), mit einem verdrehbaren glatten Schaft (1), an den eine gerade Verankerungsschraube (2) anschließt, bei deren Herausschrauben ein Mitnehmer die Axialbewegung aber nicht die Drehbewegung auf das Knochensegment (3) überträgt, **dadurch gekennzeichnet, dass** als Mitnehmer für das Knochensegment (3) eine mit einem offenen Schlitz (13) versehene Druckplatte (6) vorgesehen ist, welche durch Einschieben orthogonal zur Längsrichtung des Schaftes (1) an diesem in Axialrichtung festlegbar ist.

2. Einrichtung nach Anspruch 1, **dadurch gekennzeichnet, dass** der Durchmesser des Schaftes (1) nicht kleiner ist als der der Verankerungsschraube (2).

3. Einrichtung nach Anspruch 1 oder 2, **dadurch gekennzeichnet, dass** zur Festlegung der Druckplatte (6) am Schaft (1) eine umlaufende Rinne (9) vorgesehen ist.

4. Einrichtung nach einem der Ansprüche 1 bis 3, **dadurch gekennzeichnet, dass** die Druckplatte (6) als Hilfe beim Herausziehen eine Bohrung (10) aufweist.

5. Einrichtung nach einem der Ansprüche 1 bis 4, **dadurch gekennzeichnet, dass** die Druckplatte (6) einen aufgebogenen Rand (11) aufweist.
